# EUROPEAN PATENT APPLICATION

(11) **EP 4 085 935 A1**
(43) Date of publication of application: **09.11.2022**
(21) Application number: 22169176.9
(22) Date of filing: 21.04.2022
(51) Int. Cl.: A61L 2/08, B67C 7/00

(54) **STERILIZER**

(30) Priority: 23.04.2021 JP 2021073417
(71) Applicant: Shibuya Corporation, Ishikawa 920-8681 (JP)
(72) Inventor: NISHINO, Yukinobu, Kanazawa-shi, Ishikawa, 920-8681 (JP); NISHI, Tokuo, Kanazawa-shi, Ishikawa, 920-8681 (JP); YAMAMOTO, Yukihiro, Kanazawa-shi, Ishikawa, 920-8681 (JP); EDA, Masaaki, Kanazawa-shi, Ishikawa, 920-8681 (JP); OOKURA, Takashi, Kanazawa-shi, Ishikawa, 920-8681 (JP); YASUDA, Hiroto, Kanazawa-shi, Ishikawa, 920-8681 (JP)
(74) Representative: Hoffmann Eitle

(57) **Abstract**

A sterilizer, comprising an article-transporting means for holding articles by article-holding members and transporting the articles; a sterilizing chamber housing the article-transporting means; a radiation emission unit having an irradiation window for emitting radiation to the article; an interval-altering means for altering a conveyance interval of the articles delivered to the article-transporting means, and the interval-altering means is provided on the upstream side of the article-transporting means; and a controller for controlling the operation of the sterilizer; wherein the controller can switch drive mode between a first-sterilizing drive mode and a second-sterilizing drive mode; in the first-sterilizing drive mode, the interval-altering means delivers the articles to the article transport means at an interval that is an integral multiple of the interval of the article holding members; and in the second-sterilizing drive mode, the interval-altering means delivers the articles to the article transport means at an interval that is equal to the interval of the article-holding members.

## Description

### BACKGROUND OF THE INVENTION

### 1. Field of the Invention

The present invention relates to a sterilizer that sterilizes moving articles with radiation such as electron beams, ultraviolet rays and the like.

### 2. Description of the Related Art

An electron beam sterilizer, which is one example of a radiation sterilizer, irradiates articles continuously transported through a sterilizing chamber with electron beams to sterilize the articles. The articles are transported by grippers provided along the outer circumference of a rotating wheel at a predetermined pitch. An article held by a gripper and transported across the sterilizing chamber is irradiated with electron beams through the irradiation window of an electron beam irradiation unit. Namely, the articles are exposed to electron beams and sterilized while being rotated by the rotating wheel through a predetermined area (irradiation area) facing the irradiation window, as disclosed in Japanese Patent No. 5621567.

### SUMMARY OF THE INVENTION

Examples of product lines sterilized with the electron beam sterilizer include a filling line for a container such as a PET bottle. In such a filling line, the production capacity (supply amount) differs depending on the size of the article due to the difference in the filling capacity and the like. For example, the production capacity of a small-capacity container per unit time is higher than that of a large-capacity container. Therefore, when the electron beam sterilizer is applied to a line that handles articles having different processing capacities depending on the type of article, it is necessary to change the transport speed of the rotating wheel according to the type of the article. On the other hand, the absorbed dose per article in the electron beam sterilizer varies depending on the output energy of the electron beam and the article's time spent in the irradiation area.

For example, when the sterilizer is used in both the 'A' mode for processing a 500ml container at a processing speed of 600 bpm and the 'B' mode for processing a 2L container at a processing speed of 300 bpm, the grippers of the rotating wheel in the conventional sterilizer are spaced at intervals suitable for large containers so that adjacent containers do not interfere with each other. Therefore, the interval of the grippers on the rotating wheel is adjusted to an interval suitable for the 'B' mode, for example, a 200mm pitch. And when the distance traveled in the electron beam irradiation area is 1m, each container in the 'B' mode will stay in the irradiation area for about 2 seconds. On the other hand, in the case of the 'A' mode, the transport speed needs to be set twice that of the 'B' mode, so that each container in the 'A' mode will stay in the irradiation area only for about 1 second. Therefore, in order to maintain an appropriate absorption dose in the 'A' mode, it is necessary to increase the output energy of the electron beam in the 'A' mode.

For a high-capacity conventional filling line that sterilizes both small and large-diameter containers with the same sterilizer, it is necessary to increase the output energy of the electron beam for the small containers, which increases the running cost.

One aspect of the present invention is a sterilizer capable of handling articles having different processing capacities while suppressing the output energy of radiation.

The primary aspect of the present invention is a sterilizer comprising an article-transporting means for holding articles by article-holding members and transporting the articles; a sterilizing chamber housing the article-transporting means; an radiation emission unit having an irradiation window for emitting radiation to the an article; an interval-altering means for altering a conveyance interval of the articles delivered to the article-transporting means, the interval-altering means provided on the upstream side of the article-transporting means; and a controller for controlling the operation of the sterilizer. The controller can switch drive mode between a first-sterilizing drive mode and a second-sterilizing drive mode. In the first sterilizing drive mode, the interval-altering means delivers the articles to the article transport means at an interval that is an integral multiple of the interval of the article holding members. In the second-sterilizing drive mode, the interval-altering means delivers the articles to the article transport means at an interval that is equal to the interval of the article holding members.

### BRIEF DESCRIPTION OF THE DRAWINGS

The objects and advantages of the present invention will be better understood from the following description with references to the accompanying drawings in which:
Fig. 1 is a plan view illustrating an arrangement of sterilizer of an embodiment of the present invention;
FIG. 2 is a plan view illustrating the arrangement of a first pitch altering wheel, an inlet wheel and a first intermediate wheel with the first pitch altering wheel at the center;
Fig. 3 is a plan view illustrating a transport operation of article in a first-sterilizing drive mode; and
Fig 4 is a plan view illustrating a transport operation of article in a second-sterilizing drive mode.

### DESCRIPTION OF THE PREFERRED EMBODIMENTS

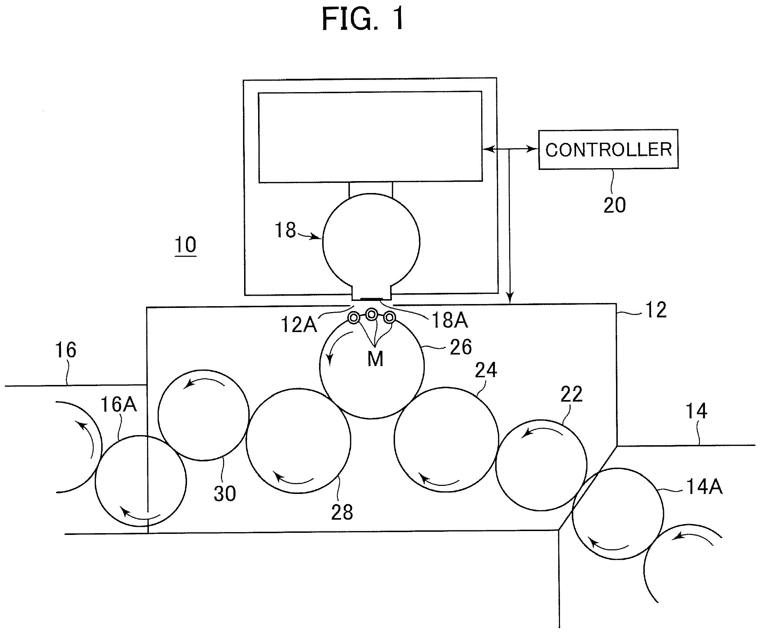

The present invention is described below with references to the embodiments shown in the drawings. Fig. 1 is a plan view illustrating configurations of a sterilizer of an embodiment of the present invention.

An exemplary sterilizer of the present embodiment is an electron beam sterilizer 10. The electron beam sterilizer 10 handles articles M supplied in different quantities per unit time (processing capacities.) The article M may be a container in which processing such as blow molding is performed upstream of the electron beam sterilizer 10. The electron beam sterilizer 10 handles containers having different capacities. The electron beam sterilizer 10 includes a sterilizing chamber 12. The article M is supplied from an inlet chamber 14 into the sterilizing chamber 12, and is discharged from the discharge chamber 16 after undergoing sterilization treatment in the sterilizing chamber 12 is completed.

An electron beam irradiation unit (a radiation emission unit) 18 is arranged adjacent to the sterilizing chamber 12. On the side surface of the sterilizing chamber 12, an opening 12A is provided through which the electron beam is emitted from the irradiation window 18A into the sterilizing chamber 12 at a position corresponding to the irradiation window 18A of the electron beam irradiation unit 18. The output energy of the electron beam irradiation unit 18 can be controlled by the control device 20.

A plurality of rotating wheels are arranged in the sterilizing chamber 12 and each wheel is provided with a plurality of grippers (article-holding members) along the outer periphery thereof. The article M is delivered between the grippers of each rotating wheel and conveyed in the sterilizing chamber 12. The sterilizing chamber 12 includes a first pitch altering wheel (interval-altering means) 22, a first intermediate wheel (article transporting means) 24, a transfer wheel (article transporting means) 26, a second intermediate wheel 28, and a second pitch altering wheel (interval changing means) 30, which are arranged in this order from the upstream side.

The article M is supplied from an inlet wheel 14A inside the inlet chamber 14 to the first pitch altering wheel 22. The article M held by the second pitch altering wheel 30 is discharged from the sterilizing chamber 12 via the discharge wheel 16A in the discharge chamber 16. In the electron beam sterilizer 10, the first pitch altering wheel 22, the first intermediate wheel 24, the transfer wheel 26, the second intermediate wheel 28, and the second pitch altering wheel 30 are rotated in synchronization with each other, and their rotations are controlled by the controller 20.

The transfer wheel 26 is arranged adjacent to the opening 12A so that the gripper passes through an area (irradiation area) in the sterilizing chamber 12 to which the electron beam is radiated. That is, the article M transported by the transfer wheel 26 passes through the irradiation area during transport such that the article M is exposed to the electron beam and sterilized in this area.

In the present embodiment, an interval (conveyance pitch) P1 between the grippers of the transfer wheel 26 and the first and second intermediate wheels 24 and 28 is fixed to the same interval. The above conveyance pitch is set at intervals suitable for the article M supplied at the fastest supply speed (highest processing capacity) among the articles M handled by the electron beam sterilizer 10. Furthermore, in the present embodiment, a gripper interval (conveyance pitch) P2 is applied when handling a small container. The conveyance pitch P2 may be set to twice the conveyance pitch P1. On the other hand, the gripper interval (conveyance pitch) P3 of the wheels on the upstream side of the first pitch altering wheel 22 such as the introduction wheel 14A and the wheels on the downstream side the second pitch altering wheel 30 such as the discharge wheel 16A is determined according to processing devices provided on the upstream and downstream side.

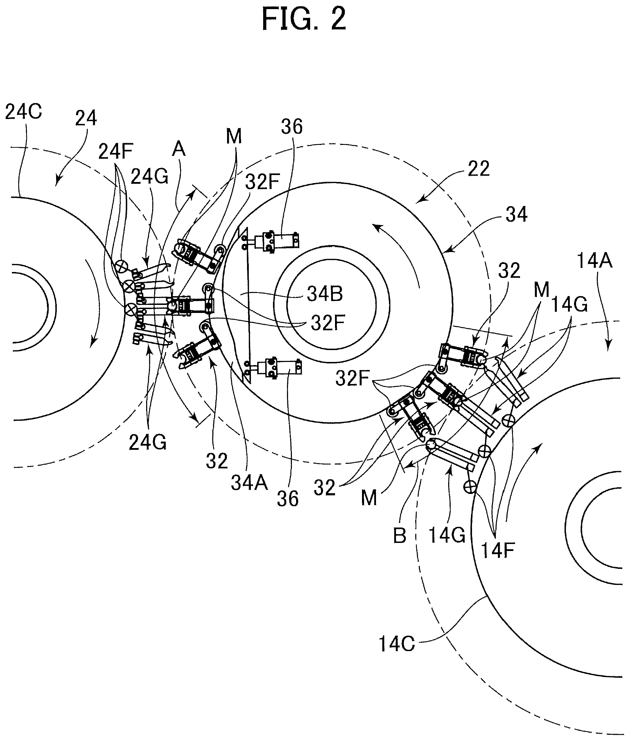

The first and second pitch-altering wheels 22 and 30 have substantially the same configuration. As will be described later with reference to FIG. 2, an interval (conveyance pitch) P between the grippers 32 of the first and second pitch-altering wheels 22 and 30 can be set to the interval P1 or P2 in a delivery section A where the article M is delivered to the first intermediate wheel 24 and delivered from the second intermediate wheel 28. Furthermore, the conveyance pitch P can be set to the interval P3 in the delivery section B where the article M is delivered from the introduction wheel 14A and delivered to the discharge wheel 16A. The first pitch altering wheel 22 of the present embodiment may switch the interval P of the grippers 32 between intervals P1 and P2 only in the delivery section A where the article M is delivered to the first intermediate wheel 24. On the other hand, the interval P is set to P3 in other sections including the delivery section B. The interval (conveyance pitch) of the grippers 32 in the delivery section A is selected according to the processing capacity of the article to be handled.

FIG. 2 is a plan view showing the arrangement of the first pitch altering wheel 22, the inlet wheel 14A and the first intermediate wheel 24 with the first pitch altering wheel 22 at the center, where the inlet wheel 14A and the first intermediate wheel 24 are partially illustrated. As shown in FIG. 2, the grippers 14G and 24G of the inlet wheel 14A and the first intermediate wheel 24 are opened and closed by engagement of cam followers 14F, 24F and cams 14C, 24C, with the cam followers 14F and 24F being provided at the tips of levers attached to the rotation shafts of each gripper. The gripper 32 of the first pitch-altering wheel 22 does not have an opening/closing mechanism. Instead, the gripper 32 is configured so that the article M (e.g., the neck portion of a PET bottle) can be pushed between the pair of grip members attracted by the spring and held between the grip members. Accordingly, each of the grippers 14G, 32, and 24G grips the article M in the section other than sections A and B, and delivers the article M in the sections A and B. Incidentally, because of the grippers of the transfer wheel 26, the second intermediate wheel 28, the second pitch-altering wheel 30, and the discharge wheel 16A are opened and closed by the same mechanism, the description thereof will be omitted.

The cam 34 of the first (second) pitch-altering wheel 22 (30) includes a first movable cam 34A and a second movable cam 34B in section A. The first and second movable cams 34A and 34B can be displaced in the radial direction by a linear actuator 36, such as an air cylinders or the like. When one movable cam is pushed outwards and functions as a cam, the other movable cam is retracted into the standby position. Namely, the orientation of the gripper 32 and the swing of the cam follower 32F are controlled by the cam 34 in any section other than section A, and in section A they are controlled by either the first movable cam 34A or the second movable cam 34B.

In the sections other than section A, the gripper 32 is moved at the interval P3 so that the interval P3 is maintained in section B. When the first movable cam 34A is pushed out in the section A, the interval of the grippers 32 is switched to P2 while the grippers 32 are moving in section A. On the other hand, when the second movable cam 34B is pushed out, the interval of the grippers 32 is switched to P1 while the grippers 32 are moving in section A. In FIG. 2, the linear actuator 36 of the first movable cam 34A is arranged below the linear actuator 36 of the second movable cam 34B. Each linear actuator 36 is controlled by the controller 20.

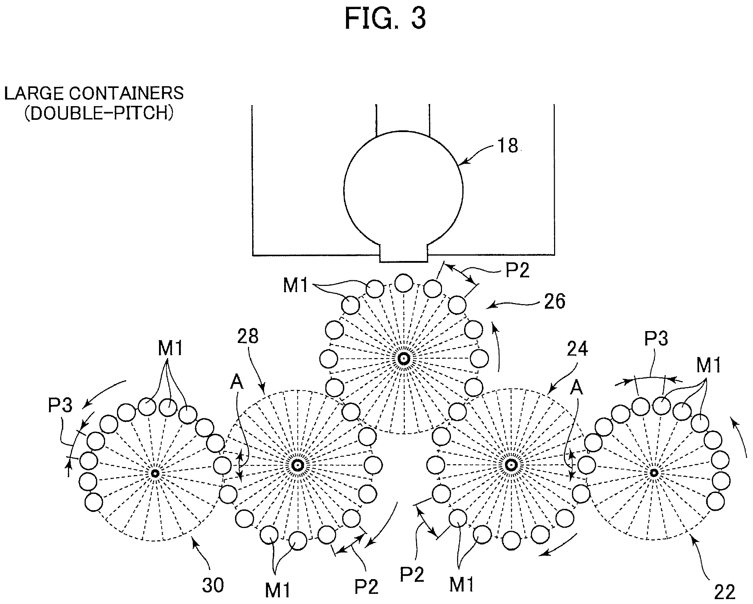

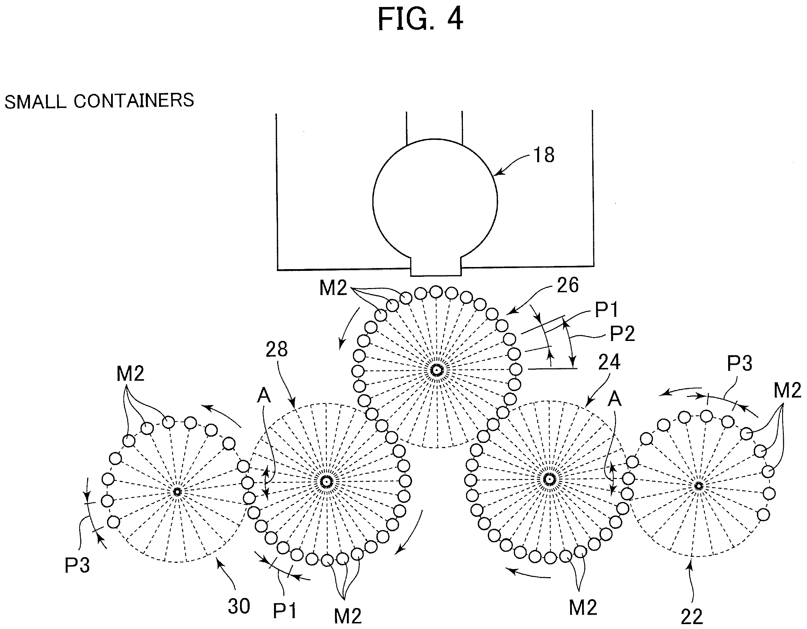

Next, with reference to FIGS. 3 and 4, a transport operation in a first-sterilizing drive mode for sterilizing a large-capacity article M1 and a transport operation in a second-sterilizing drive mode for sterilizing a small-capacity article M2 will be described. In the present embodiment, the article M1 is a large container and the article M2 is a small container.

As shown in FIG. 3, when the article M1 is sterilized, the electron beam sterilizer 10 is set to the first-sterilizing drive mode. In the first-sterilizing drive mode, the first movable cam 34A is selected (pushed out) so that the wheels 24, 26, 28 transport the article M1 at the conveyance pitch P2. The wheels 22, 24, 26, 28, and 30 are each rotated synchronously at the rotation speed corresponding to article M1. The electron beam irradiation unit 18 emits an electron beam with an intensity corresponding to that of article M1. Note that in the first-sterilizing drive mode, every other gripper of each of the first and second intermediate wheels 24, 28 and transfer wheel 26 grips the article M1 so that the article M1 is transported at the conveyance pitch P2.

On the other hand, as shown in FIG 4, the electron beam sterilizer 10 is set to the second-sterilizing drive mode when sterilizing the article M2. In the second-sterilizing drive mode, the second movable cam 34B is selected (pushed out). Thereby, the first pitch-altering wheel 22 receives the article M2 from the inlet wheel 14A at the conveyance pitch P3 and switches the pitch to P1 from P3. The first pitch-altering wheel 22 then delivers the article M2 to the first intermediate wheel 24. At the same time, the second pitch-altering wheel 30 receives the article M1 from the second intermediate wheel 28 at the conveyance pitch P1 and switches the pitch to P3 from P1. The second pitch altering wheel 30 then delivers the article M2 to the discharge wheel 16A. The wheels 22, 24, 26, 28, and 30 are each rotated synchronously at the rotation speed corresponding to the article M2. The electron beam irradiation unit 18 emits the electron beam with an intensity corresponding to that of the article M2. In the first and second intermediate wheels 24 and 28 and the transfer wheel 26, all the grippers grip the article M2, and the article M2 is transported at the conveyance pitch P1.

As described above, in the present embodiment, the conveyance pitch in the article sterilizer can be changed according to the article processing capacity (article supply amount per unit time). Thereby, the time spent in the electron beam irradiation area by the articles can be shortened due to the prevention of an increase in the transport speed even when an article having a high processing capacity is handled. That is, even when an article corresponding to a high processing capacity is handled, it is possible to set a relatively long time for the article to move through the irradiation area. As a result, the output energy of the electron beam can be suppressed while maintaining sufficient sterilization of the article even when the article is supplied at a high processing capacity.

In the first and second pitch-altering wheels of the present embodiment, the gripper interval (conveyance pitch) can be switched between P1 and P2, which is twice P1. However, the conveyance pitch may be further switched to an integral multiple of three times or more of P1. In such case, the interval (conveyance pitch) can be selected by applying three or more movable cams. Furthermore, in the present embodiment, the article transportation mechanism utilizing the rotating disk provided with the grippers has been described as an example. However, the mechanism and configuration for holding and transporting the article are not limited to the present embodiment. Furthermore, the radiation applied in sterilization is not limited to electron beams, but may be non-ionizing radiation such as ultraviolet rays or electromagnetic waves such as gamma rays, or other particle beams.

Although the embodiments of the present invention have been described herein with reference to the accompanying drawings, obviously many modifications and changes may be made by those skilled in this art without departing from the scope of the invention.

## Claims

1. A sterilizer, comprising:
an article-transporting means for holding articles by article-holding members and transporting the articles;
a sterilizing chamber housing the article-transporting means;
a radiation emission unit having an irradiation window for emitting radiation to the article;
an interval-altering means for altering a conveyance interval of the articles delivered to the article-transporting means, and the interval-altering means is provided on the upstream side of the article-transporting means; and
a controller for controlling the operation of the sterilizer;
wherein the controller can switch drive mode between a first-sterilizing drive mode and a second-sterilizing drive mode;
in the first-sterilizing drive mode, the interval-altering means delivers the articles to the article-transport means at an interval that is an integral multiple of the interval of the article-holding members; and
in the second-sterilizing drive mode, the interval-altering means delivers the articles to the article transport means at an interval that is equal to the interval of the article-holding members.
